# EUROPEAN PATENT SPECIFICATION

(11) **EP 1 082 939 B1**
(45) Date of publication and mention of the grant of the patent: **02.11.2005**
(21) Application number: 00302063.3
(22) Date of filing: 14.03.2000
(51) Int. Cl.: A61B 3/08, H04N 13/00

(54) **Digital synoptophore**
Digitales Synoptophor
Synoptophore digital

(30) Priority: 01.09.1999 GB 9920477
(43) Date of publication of application: 14.03.2001
(73) Proprietor: Assaf, Ahmed A., Dr., Winslow, Bucks., MK18 3RH (GB)
(72) Inventor: Assaf, Ahmed A., Dr., Winslow, Bucks., MK18 3RH (GB)

(56) References cited:
- EP-A- 0 319 446
- WO-A-98/05251
- FR-A- 1 198 064
- GB-A- 2 146 877
- GB-A- 2 332 271
- US-A- 2 091 173
- KUSUBE T. ET AL: 'VEP ASSESSMENT OF SUPPRESSION IN PATIENTS WITH EXTROPIA' FOLIA OPHTHALMPOL. JPN vol. 40, 1989, pages 1975 - 1980, XP009020325

## Description

This invention relates to functional and motor assessment of strabismus (squint) patients.

The synoptophore is used currently in assessing strabismus patients. Additionally, it can be used to measure the angle of deviation and treating binocular anomalies by orthoptic exercises.

WO98/05251 discloses in figure 2a and page 3 line 6-23 a synoptophore comprising left and right optical systems in the form of a tube coupled with a single viewing screen 142. The device is configured such that each eye sees only one image.

US2091173 discloses in figures 1 and 19 an orthoptic training apparatus comprising a single display stand with movable targets wherein each eye sees only a portion of the display.

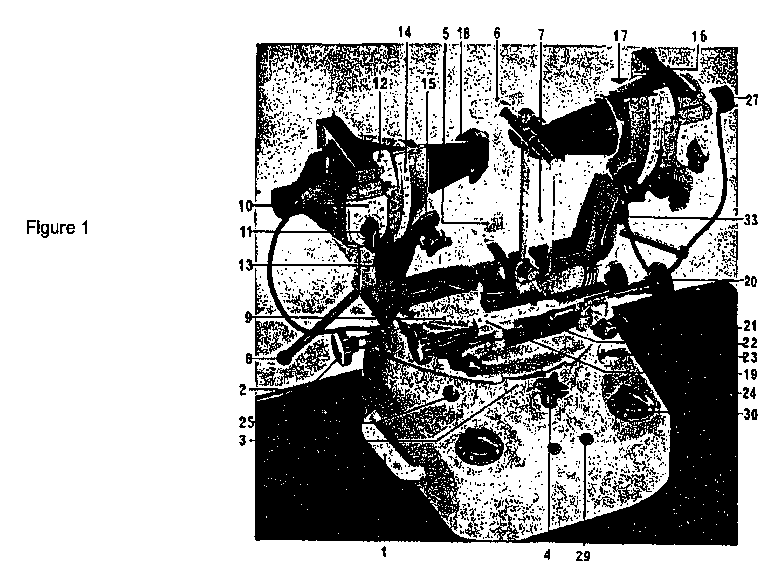

Current synoptophores (figures 1) are electrical and essentially consist of 2 columns; each is used to project an image for viewing by each eye separately. The synoptophore is used to perform the following tests on patients with strabismus:
1. Simultaneous perception (foveal, macular and para-macular)
2. Fusion
3. Stereopsis
4. Assessing retinal correspondence
5. After image testing
6. Measure the angle of deviation
7. Orthoptic exercises

Such tests are used in every day practice in assessing strabismus patients. Images in the current synoptophore are obtained by manually changing different slides in each tube to perform various functions. The current invention as defined in claim 1 utilises computer technology in the production of the test images on a computer display screen.
- Figure 1: is a picture of a prior art synoptophore.
- Figure 2: shows a digital synoptophore according to the invention.
- Figure 3: shows a diagram of the arms of the digital synoptophore according to the invention.

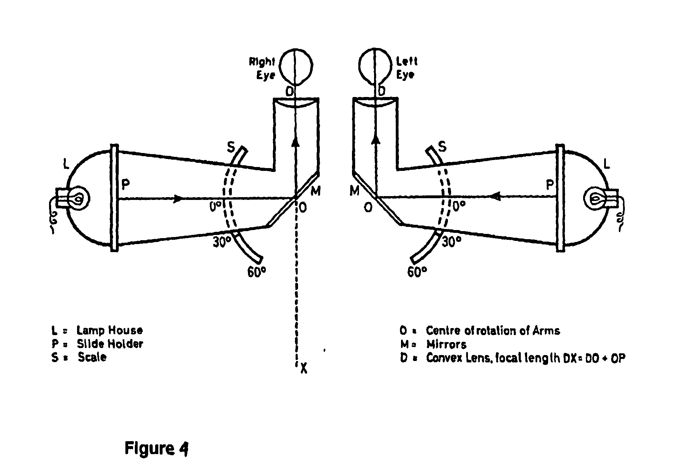

- Figure 4: shows a diagram of the arms of a prior art synoptophore.
- Figure 5: depicts samples of pictures to be used in the testing process.

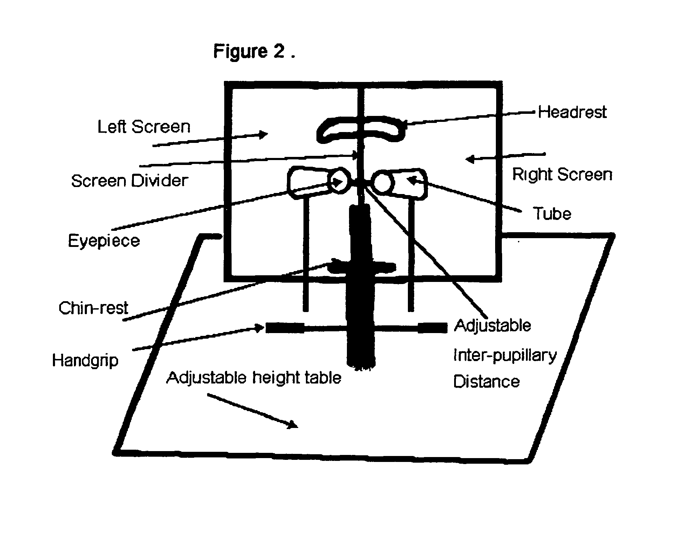

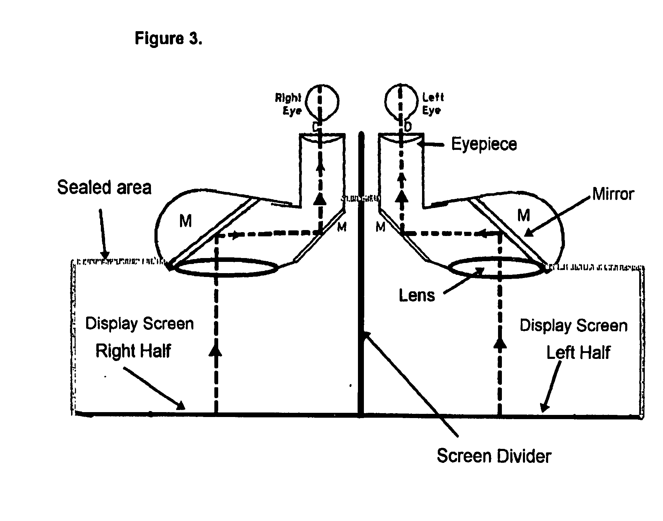

This invention consists of both hardware and software components:
1) Adjustable chin-rest and headrest.
2) Computer with display screen (with computer)
3) A opaque vertical screen divider, to divide the display screen into 2 equal right and left halves (figure 2).
4) An input device to move the generated screen targets, such as mouse, trackball ... etc.
5) A tube to carry the images from the display screen to each eye separately. At the medial end of the tube there is an eyepiece and at the lateral end there is a lens (figure2 and figure 3).
*6)* The length of each tube varies with the screen size, but both are equal in length. The medial end of each tube is sliding to allow for inter-pupillary distance (IPD) adjustment but the *lateral end is fixed and it's centre corresponds to the centre of each half of the screen.*
7) At the lateral end of each tube is a lens to focus images from the display screen on the patient retina. The power of the lens in the eyepiece will vary depending on the size of the screen and testing distance. The screen should lie at the primary focal point of the lens so as rays leaving the lens to the patient's eye should be parallel to prevent patient accommodation. An additional +3.00 lens will be used for near testing. For example, a screen size of 280 mm wide and 210 mm high (15 inch monitor) would produce two half screens of 140 mm wide x 210 mm high. The testing distance will be 11.7 cm and at this distance this screen size will allow eccentric testing at 15 degrees away from the centre. A lens placed at the distal end of the tube would be +8.50 diopters, but the required lens power will be less if the lens is placed in the eyepiece.
8) The eyepieces at the medial end of the tube, one for each eye, are interchangeable.
9) Prisms for the eyepieces.
10) Polarised lens, iris diaphragm, occluder, and red and blue filters for eyepieces.
11) Software.

This unit can be sealed or left open. The seal will extend between the tubes and including the display screens and the screen divider (see figure 3).

Instead of mirrors as in figure 3 inverting prisms can be used.

### TESTING METHODS

Before examination the inter-pupillary distance (IPD) for the patient must be first measured in millimetres. The distance between the eyepieces is then set according to this measurement. The child places his chin on the chin-rest and his forehead on the headrest. The Chin-rest is adjusted so the eye level is at the centre of screen vertically. Additionally, each eye is separated each distance, i.e. the screen divider is ½ way between the pupillary centre of the 2 eyes.

### I) Sensory and motor assessment of strabismus patients:

Objects are generated at each half of the screen to test various visual functions as above. These objects are of 3 sizes: 10, 5 and 3 degrees to test para-macular, macular and foveal functions.

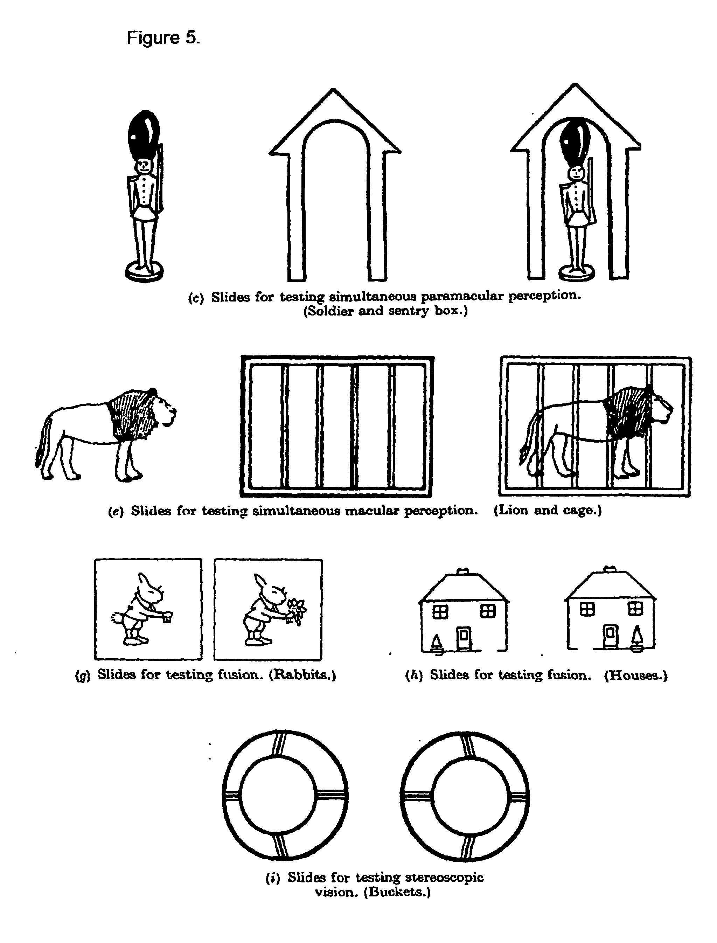

The computer generates two complementary images one in each half of the display screen, examples as in figure 5. A child with strabismus viewing the 2 halves of the screen, through the eyepieces, will see the images with a different relationship from that of normal.

Images generated in each half of the screen can independently be moved laterally, vertically, or rotated along the antero-posterior axis for the measurement of horizontal, vertical, and torsional deviation correspondingly.

The child is instructed to move one image as above using an input device so the relationships reach the intended end point (see figure). Background targets to simulate distant fixation, upon which the above images are generated, can be used to prevent instrument convergence.

Beside this, stereopsis and binocular functions can be tested by using different displays.

Additionally, each half can be switched of(or blackened), flashed, or light intensity changed to perform various visual function tests.

### II) Measurement of angle of strabismus in 9 positions of gaze:

Fixation targets (as described above) can be generated at 15 or 30 degrees away from the centre of fixation of each eye (depending on the size of the screen). This can be used for the measurement of the deviation in the 9 positions of gaze.

## Claims

1. A digital synoptophore comprising a computer controlled display device, said computer controlled display device comprising a computer system, an input device, display means and software configured to assess strabismus and to carry out orthoptic exercises, adjustable chin- and head-rests;
**characterized in that**
said display means comprise a single monitor with a screen divider and two tubes one on either side, wherein each of said tubes is configured to carry the images from the display screen to each eye separately; and wherein each of said tubes includes an eyepiece at the medial end of the tube and a lens at the lateral end, and; wherein the medial end of each of said tubes is slidable to allow for inter-pupillary distance adjustment and the lateral end is fixed in a position where its centre is aligned with the centre of each half of said screen.

2. A digital Synoptophore as claimed in Claim 1 wherein images used for testing are generated on each half of the screen independently.

3. A digital Synoptophore as claimed in Claim 2 wherein the images in each half can be moved independently by the patient horizontally, vertically, and rotated along the antero-posterior axis.

4. A digital Synoptophore as claimed in Claim 3 wherein other features include blackening of each half' of the screen, flashing and changing intensity.

5. A digital Synoptophore as claimed in Claim 4 wherein this device is configured to assess sensory and motor functions of strabismus patients and to measure deviation angles centrally and at eccentric position of gaze.

## Patentansprüche

1. Ein digitales synoptophone,das einen Computer enthält und ein gesteuertes Sichtanzeigegerät, das besagter Computer gesteuerte Sichtanzeigegerät und ein Computer system ist enthalten, ein Eingabegerät, Anzeige Mittel und Software, die zusammengebaut wurden, um Strabismus festzusetzen und orthoptic Übungen ausführen kann und vorstellbares Kinn- und Kopfreste, **gekennzeichnet** in diese besagten Austellung Mitteln einen einzelnen Monitor mit einem Schirmteiler und zwei Röhren einer auf beiden Seiten, worin jede besagte Röhre zusammen gebaut wird, um die Bilder vom Bildschirm zu jedem Auge separat zu tragen, und worin jedes der besagten Röhre ein Okular enthält am Mittelende der Röhre und ein Objektiv am seitlichen Ende worin das Mittelende von jeder besagten Röhre verschiebbar ist und ermöglicht einen zwischen - papillary Abstand Justage und das seitliche Ende in einer Position geregelt wird, in der seine Mitte mit der Mitte jeder Hälfte des besagten Bildschirmes ausgerichtet ist.

2. Ein digitales synoptophore wie behauptet in Anspruch 1, worin die Bilder, die für Prüfung verwendet werden, auf jeder Hälfte des Bildschirmes unabhängig erzeugt werden.

3. Ein digitales synoptophore wie behauptet in Anspruch 2, worin die Bildnisse in jeder Hälfte von Patienten unabhängig horizontal, vertical verschoben werden kann und entlang der anteroposteriore Mittellinie/Achse gedreht werden können.

4. Ein digitales synoptophore wie behauptet in Anspruch 3, worin andere Eigenschaften wie das Schwärzen jeder Hälfte des Bildschirmes, blitzend und wechselnde Intensität mit eingeschlossen sind.

5. Ein digitales synoptophone wie behauptet in Anspruch 4 worin diese Vorrichtung , um die sensorische und Bewegungsfunktionen der strabismus Patienten zusammengebaut wird, Abweichungwinkel zentral zu messen und die Exzenterposition des Blicks festzusetzen.

## Revendications

1. Synoptophore numérique, comprenant un écran commandé par ordinateur, dénommé écran commandé par ordinateur, comprenant un système informatique, un périphérique d'entrée, des moyens d'affichage, et un logiciel configuré pour évaluer le strabisme et effectuer des exercices orthoptiques, des supports pour menton et tête, **caractérisé en ce que** les dits moyens d'affichage comprennent un moniteur avec séparateur d'écran et deux tubes, un de chaque côté, chaque tube étant configuré pour transmettre les images de l'écran d'affichage vers chaque oeil séparément, et dont chacun des dits tubes comprennent un oculaire situé à l'extrémité médiane du tube et une lentille à l'extrémité latérale, et dont l'extrémité médiane de chacun des dits tubes peut coulisser pour permettre le réglage de la distance interpupillaire, et dont l'extrémité latérale est fixée en position avec son centre aligné avec le centre de chaque moitié du dit écran.

2. Synoptophore numérique, selon la revendication 1, dans lequel les images utilisées pour le test sont produites indépendamment sur chaque moitié de l'écran.

3. Synoptophore numérique, selon la revendication 2, avec lequel le patient peut indépendamment déplacer chaque moitié horizontalement, verticalement, et la faire pivoter dans l'axe antéropostérieur.

4. Synoptophore numérique, selon la revendication 3, comprenant d'autres caractéristiques telles que le noircissement de chaque moitié de l'écran, le clignotement et le changement d'intensité.

5. Synoptophore numérique, selon la revendication 4, dont le dit appareil est configuré pour évaluer les fonctions sensorielles et motrices chez les patients souffrant de strabisme et pour mesurer les angles de déviation en position centrale et excentrique par rapport au regard.
